# EUROPEAN PATENT SPECIFICATION

(11) **EP 3 150 237 B1**
(45) Date of publication and mention of the grant of the patent: **17.04.2019**
(21) Application number: 16197104.9
(22) Date of filing: 15.10.2009
(51) Int. Cl.: A61L 31/10, A61L 31/14, A61L 31/16, A61L 31/04, A61L 31/12

(54) **HEMOSTATIC IMPLANT**
HÄMOSTATISCHES IMPLANTAT
IMPLANT HÉMOSTATIQUE

(30) Priority: 17.10.2008 US 196543 P; 05.10.2009 US 573176
(43) Date of publication of application: 05.04.2017
(62) Divisional of application: 14184313.6
(73) Proprietor: Covidien LP, Mansfield, MA 02048 (US)
(72) Inventor: BENNETT, Steven, Cheshire, Connecticut 06410 (US)
(74) Representative: Birtle, Zoë Elizabeth

(56) References cited:
- US-A- 5 201 745
- US-A1- 2002 106 409
- US-A1- 2003 031 697
- US-A1- 2004 106 344

## Description

### BACKGROUND

### Technical Field

The present disclosure relates to implants and more particularly to hemostatic implants which include a porous substrate having a first hydrogel precursor and a second hydrogel precursor applied thereto.

### Background of Related Art

In situ hemostatic therapy has primarily focused on the transformation of precursor solutions into solids within a patient's body. Transformations have been achieved by a variety of means, including precipitation, polymerization, crosslinking, and desolvation. However, significant limitations exist when using solutions for in situ hemostatic therapy. Solutions of low viscosity may flow away and be cleared from an application site before transformation and solidification occurs. Furthermore, formulation of the solutions may be complex, as preparation of precursor solutions typically requires reconstitution of the precursors, or, when the solutions are stored frozen, thawing.

US 2004/0106344 describes a haemostatic wound dressing comprising a fabric comprising a biocompatible polymer, wherein the fabric substrate comprises fibres , and a porous, polymeric matrix distributed on the first and second surface of the fabric and through said fabric. The porous, polymeric matrix comprises a biocompatible, water-soluble or water-swellable proteinaceous polymer.

US 2002/0106409 describes a hydrogel system comprising two dry precursors which form crosslinks in situ upon exposure to an aqueous physiological environment. The hydrogel system can be provided in the form of a patch, where the precursors are applied on a backing material. The precursors can be provided in separate layers on top of each other.

US 2003/0031697 describes an implant with a porous surface layer. A crosslinkable macromer system comprising a polymerization initiator mixed with polymers having pendent polymerizable groups is applied onto this surface and/or filling in the voids of the surface. The system is polymerized after implanting the article within the tissue, preferably by photo-polymerization.

Therefore it would be desirable to provide in situ hemostatic therapy which includes implantable devices combined with dry materials that are activated by the presence of aqueous physiological fluids. The combination of an implantable device with dry materials ensures the in situ hemostatic therapy will occur at the site of implantation.

### SUMMARY

An implant for use as a hemostat is provided. The implant includes a porous fibrous substrate having pores over at least a portion thereof; a first hydrogel precursor applied to a first portion of the porous substrate; and
a second hydrogel precursor applied to a second portion of the porous substrate, the first portion of the porous substrate being spatially separated from the second portion of the porous substrate, wherein the first hydrogel precursor and the second hydrogel precursor react to form a hydrogel when exposed to physiologic fluid. In embodiments, at least one of the first or second hydrogel precursors is applied to the porous substrate as a film. In embodiments, the first and second hydrogel precursors are prevented from reacting with each other until the implant is placed at the site of implantation and exposed to the physiological fluids of a patient. In embodiments, exposure of the implant to physiological fluids causes the first hydrogel precursor to migrate from the first portion of the porous substrate towards the second portion of the porous substrate and react with the second hydrogel precursor. In embodiments, the present implants display not only hemostatic properties but further display anti-adhesive properties on portions of the coated porous substrate.

### BRIEF DESCRIPTION OF THE DRAWINGS

The accompanying drawings, which are incorporated in and constitute a part of this specification, illustrate embodiments of the disclosure and, together with a general description of the disclosure given above, and the detailed description of the embodiments given below, serve to explain the principles of the disclosure.
Figures 1A-D schematically show the application of first and second hydrogel precursors to a porous substrate as described in at least one of the embodiments in the present disclosure;
Figure 2 schematically shows a variation of the embodiment shown in Figures 1A - 1C ;
Figure 3 schematically shows another variation of the embodiment shown in Figures 1A - 1C;
Figures 4A-C schematically show the application of a first hydrogel precursor to a porous substrate as described in at least one of the embodiments in the present disclosure;
Figures 5A-C schematically show the application of particles including a second hydrogel precursor to a porous substrate already having a first hydrogel precursor applied thereto as described in at least one of the embodiments in the present disclosure;
Figures 6A-C schematically show the application of a film containing a second hydrogel precursor to a porous substrate already having a first hydrogel precursor applied thereto as described in at least one of the embodiments in the present disclosure;
Figures 7A-B schematically show the simultaneous formation of a foam containing a first hydrogel precursor and a foam porous substrate; and
Figures 8A-C schematically show the application of particles including a second hydrogel precursor to a porous substrate already having a first hydrogel precursor applied thereto as described in at least one of the embodiments in the present disclosure;
Figures 9A-C schematically show the application of a film containing a second hydrogel precursor to a porous substrate already having a first hydrogel precursor applied thereto as described in at least one of the embodiments in the present disclosure;
Figures 10 schematically shows a knitted fibrous porous substrate having particles including a first hydrogel precursor applied to a first portion thereof and a film containing a second hydrogel precursor applied to second portion thereof as described in at least one of the embodiments in the present disclosure;
Figure 11 schematically shows a knitted fibrous porous substrate having a coating including a first hydrogel precursor applied to a first portion thereof and a film containing a second hydrogel precursor applied to second portion thereof as described in at least one of the embodiments in the present disclosure; and
Figure 12 schematically shows a non-woven fibrous porous substrate having particles including a first hydrogel precursor applied to a first portion thereof and a film containing a second hydrogel precursor applied to second portion thereof as described in at least one of the embodiments in the present disclosure.

### DETAILED DESCRIPTION OF PREFERRED EMBODIMENTS

Hemostatic implants in accordance with the present disclosure include a porous substrate having a first hydrogel precursor applied to a first portion of the porous substrate and a second hydrogel precursor applied to a second portion of the porous substrate. During use, the implant is oriented with the portion to which the first hydrogel precursor is applied closer to the tissue and the portion having the second hydrogel precursor applied thereto further from the tissue. In embodiments, the first and second portions may be distinguishable from one another by the addition of contrast dyes, surface texturing, coloring or other visual cues. Upon contact with tissue, such as, for example, injured tissue, the implant will soak up physiological fluid and the first hydrogel will be dissolved by the fluid. As the fluid wicks into and migrates across the implant, it will carry the dissolved first hydrogel precursor along through the implant. Eventually, the fluid will migrate through the implant sufficiently to reach the second portion to which the second hydrogel precursor is applied, thereby dissolving the second hydrogel precursor. The first and second hydrogel precursors will then react to form a biocompatible cross linked material, thereby assisting with hemostasis. In some embodiments, the biocompatible cross linked material produced by reaction of the first and second hydrogel precursors not only provide hemostatic properties but also provide the implant with anti-adhesive properties.

The porous substrate of the implant has openings or pores over at least a portion of a surface thereof. The pores may be formed in the substrate either before or after implantation. As described in more detail below, materials for forming the porous substrate are, according to the invention, fibrous structures (e.g., knitted structures, woven structures, non-woven structures, etc.). In embodiments, the pores may be in sufficient number and size so as to interconnect across the entire thickness of the porous substrate. Woven fabrics and knitted fabrics are illustrative examples of structures in which the pores can be in sufficient number and size so as to interconnect across the entire thickness of the porous substrate. In embodiments, the pores do not interconnect across the entire thickness of the porous substrate. Fused non-woven materials are illustrative examples of structures in which the pores may not interconnect across the entire thickness of the porous substrate. In embodiments, the openings or pores are located on a portion of the surface of the porous substrate, with other portions of the porous substrate having a non-porous texture. In other embodiments, the pores may be formed after implantation in situ. The in situ pore formation may be performed using any suitable method. Some non-limiting examples include the use of contact lithography, living radical photopolymer (LRPP) systems and salt leaching. Those skilled in the art reading the present disclosure will envision other pore distribution patterns and configurations for the porous substrate.

Where the porous substrate is fibrous, the fibers may be filaments or threads suitable for knitting or weaving or may be staple fibers, such as those frequently used for preparing non-woven materials. The fibers may be made from any biocompatible material. Thus, the fibers may be formed from a natural material or a synthetic material. The material from which the fibers are formed may be bioabsorbable or non-bioabsorbable. It should of course be understood that any combination of natural, synthetic, bioabsorbable and non-bioabsorbable materials may be used to form the fibers. Some non-limiting examples of materials from which the fibers may be made include, but are not limited to poly(lactic acid), poly (glycolic acid), poly(lactide, poly(glycolide), poly(trimethylene carbonate), poly (dioxanone), poly (hydroxybutyrate), poly (phosphazine), polyesters, polyethylene terephthalate, ultra-high molecular weight polyethylene, polyethylene glycols, polyethylene oxides, polyacrylamides, polyhydroxyethylmethylacrylate, polyvinylpyrrolidone, polyvinyl alcohols, polyacrylic acid, polyacetate, polycaprolactone, polypropylene, aliphatic polyesters, glycerols, poly(amino acids), copoly (ether-esters), polyalkylene oxalates, poly (saccharides), polyamides, poly (iminocarbonates), polyalkylene oxalates, polyoxaesters, polyorthoesters, polyphosphazenes, biopolymers, polymer drugs and copolymers, block copolymers, homopolymers, blends and combinations thereof.

Where the porous substrate is fibrous, the porous substrate may be formed using any method suitable to forming fibrous structures, including but not limited to knitting, weaving, non-woven techniques, wet-spinning, electro-spinning, extrusion, co-extrusion, and the like. Suitable techniques for making fibrous structures are within the purview of those skilled in the art. In embodiments, the textile has a three dimensional structure, such as the textiles described in U.S. Patent Nos. 7,021,086 and 6,443,964.

In embodiments, the porous substrate is made from fibers of oxidized cellulose. Such materials are known and include oxidized cellulose hemostat materials commercially available under the trade name SURGICEL®. Methods for preparing oxidized cellulose hemostat materials are known to those skilled in the art and are disclosed, for example in U.S. Patent Nos. 3,364,200; 4,626,253; 5,484,913; and 6,500,777.

The porous substrate can be at least 0.1 cm thick, in embodiments from about 0.2 to about 1.5 cm thick. The size of the pores in the porous substrate can be from about 2 µm to about 300 µm, in embodiments from about 50 µm to about 150 µm. It is envisioned that the pores of the substrate may be arranged in any manner in the substrate. In still other embodiments, the pores may be configured to create a gradient in the porous substrate. The gradient may further enhance the porous substrates ability to absorb the physiologic fluid and direct the migration of the physiological fluid carrying the first hydrogel precursor towards the second hydrogel precursor.

In embodiments, the implant is a made from non-denatured collagen or collagen which has at least partially lost its helical structure through heating or any other method, consisting mainly of non-hydrolyzed α chains, of molecular weight close to 100 kDa. The term "non-denatured collagen" means collagen which has not lost its helical structure. The collagen used for the implant of present implant may be native collagen or atelocollagen, notably as obtained through pepsin digestion and/or after moderate heating as defined previously. The collagen may have been previously chemically modified by oxidation, methylation, ethylation, succinylation or any other known process. The collagen may also be cross-linked with any suitable crosslinker, such as genipin, isocyanates, and aldehydes. The origin and type of collagen may be as indicated for the non-implant described above.

The porous substrate has a first hydrogel precursor applied thereto and a second hydrogel precursor applied thereto. The terms "first hydrogel precursor" and "second hydrogel precursor" each means a polymer, functional polymer, macromolecule, small molecule, or crosslinker that can take part in a reaction to form a network of crosslinked molecules, e.g., a hydrogel.

In embodiments, at least one of the first or second hydrogel precursors is a small molecule of about 1000 Da or less, and is referred to as a "crosslinker". The crosslinker preferably has a solubility of at least 1 g/100 mL in an aqueous solution. A crosslinked molecule may be crosslinked via an ionic or covalent bond, a physical force, or other attraction.

In embodiments, at least one of the first or second hydrogel precursors is a macromolecule, and is referred to as a "functional polymer". The macromolecule, when reacted in combination with a crosslinker, is preferably at least five to fifty times greater in molecular weight than the small molecule crosslinker and can be less than about 60,000 Da. In embodiments, a macromolecule that is seven to thirty times greater in molecular weight than the crosslinker is used and, in embodiments a macromolecule that is about ten to twenty times difference in weight is used. Further, a macromolecular molecular weight of 5,000 to 50,000 is useful. The term polymer, as used herein, means a molecule formed of at least three repeating groups.

Each of the first and second hydrogel precursors is multifunctional, meaning that it comprises two or more electrophilic or nucleophilic functional groups, such that, for example, a nucleophilic functional group on the first hydrogel precursor may react with an electrophilic functional group on the second hydrogel precursor to form a covalent bond. At least one of the first or second hydrogel precursors includes more than two functional groups, so that, as a result of electrophilic-nucleophilic reactions, the precursors combine to form crosslinked polymeric products. Such reactions are referred to as "crosslinking reactions".

In embodiments, each of the first and second hydrogel precursors includes only one category of functional groups, either only nucleophilic groups or only electrophilic functional groups, so long as both nucleophilic and electrophilic precursors are used in the crosslinking reaction. Thus, for example, if the first hydrogel precursor has nucleophilic functional groups such as amines, the second hydrogel precursor may have electrophilic functional groups such as N-hydroxysuccinimides. On the other hand, if first hydrogel precursor has electrophilic functional groups such as sulfosuccinimides, then the second hydrogel precursor may have nucleophilic functional groups such as amines or thiols. Thus, functional polymers such as proteins, poly(allyl amine), styrene sulfonic acid, or amine-terminated di- or multifunctional poly(ethylene glycol) ("PEG") can be used.

The first and second hydrogel precursors may have biologically inert and water soluble cores. When the core is a polymeric region that is water soluble, preferred polymers that may be used include: polyether, for example, polyalkylene oxides such as polyethylene glycol("PEG"), polyethylene oxide ("PEO"), polyethylene oxide-co-polypropylene oxide ("PPO"), co-polyethylene oxide block or random copolymers, and polyvinyl alcohol ("PVA"); poly(vinyl pyrrolidinone) ("PVP"); poly(amino acids); poly (saccharides), such as dextran, chitosan, alginates, carboxymethylcellulose, oxidized cellulose, hydroxyethylcellulose, hydroxynethylcellulose, hyaluronic acid; and proteins such as albumin, collagen, casein, and gelatin. The polyethers and more particularly poly(oxyalkylenes) or poly(ethylene glycol) or polyethylene glycol are especially useful. When the core is small molecular in nature, any of a variety of hydrophilic functionalities can be used to make the first and second hydrogel precursors water soluble. For example, functional groups like hydroxyl, amine, sulfonate and carboxylate, which are water soluble, maybe used to make the precursor water soluble. In addition, N-hydroxysuccinimide ("NHS") ester of subaric acid is insoluble in water, but by adding a sulfonate group to the succinimide ring, the NHS ester of subaric acid may be made water soluble, without affecting its reactivity towards amine groups.

If it is desired that the biocompatible crosslinked polymer resulting from the reaction of the first and second hydrogel precursors be biodegradable or absorbable, one or more of the first and second hydrogel precursors may have biodegradable linkages present between the functional groups. The biodegradable linkage optionally also may serve as the water soluble core of one or more of the precursors. In the alternative, or in addition, the functional groups of the first and second hydrogel precursors may be chosen such that the product of the reaction between them results in a biodegradable linkage. For each approach, biodegradable linkages may be chosen such that the resulting biodegradable biocompatible crosslinked polymer will degrade, dissolve or be absorbed in a desired period of time. Preferably, biodegradable linkages are selected that degrade under physiological conditions into non-toxic products.

The biodegradable linkage may be chelates or chemically or enzymatically hydrolyzable or absorbable. Illustrative chemically hydrolyzable biodegradable linkages include polymers, copolymers and oligomers of glycolide, dl-lactide, 1-lactide, caprolactone, dioxanone, and tritnethylene carbonate. Illustrative enzymatically hydrolyzable biodegradable linkages include peptidic linkages cleavable by metalloproteinases and collagenases. Additional illustrative biodegradable linkages include polymers and copolymers of poly(hydroxy acid)s, poly(orthocarbonate)s, poly(anhydride)s, poly(lactone)s, poly(amino acid)s, poly(carbonate)s, poly(saccharide)s and poly(phosphonate)s.

In embodiments, the biodegradable linkage may contain ester linkages. Some non-limiting examples include esters of succinic acid, glutaric acid, propionic acid, adipic acid, or amino acids, as well as carboxymethyl esters.

In embodiments, a multifunctional nucleophilic polymer such as trilysine may be used as a first hydrogel precursor and a multifunctional electrophilic polymer such as a multi-arm PEG functionalized with multiple NHS groups may be used as a second hydrogel precursor. The multi-arm PEG functionalized with multiple NHS groups can for example have four, six or eight arms and have a molecular weight of from about 5,000 to about 25,000. Many other examples of suitable first and second precursors are described in U.S. Patent Nos. 6,152,943; 6,165,201; 6,179,862; 6,514,534; 6,566,406; 6,605,294; 6,673,093; 6,703,047; 6,818,018; 7,009,034; and 7,347,850.

The first hydrogel precursor is applied to a first portion of the porous substrate and a second hydrogel precursor applied to a second portion of the porous substrate. For example, the precursors may be applied in a dry form, such as particulate matter or in a solid or semi-solid state such as a film, or foam. In embodiments, at least one of the first or second hydrogel precursors is applied to the porous substrate as a film. In embodiments, the first portion of the substrate having the fist hydrogel precursor applied thereto is spatially separated from the second portion of the porous substrate having the second hydrogel precursor applied thereto. Having the first and second hydrogel precursors spatially separated from each other prevents them from reacting with each other until the implant is placed at the site of implantation and exposed to the physiological fluids of a patient.

The first hydrogel precursor may be applied to the porous substrate using any suitable method known to those skilled in the art, including, but not limited to spraying, brushing, dipping, pouring, laminating, etc. In embodiments, the first hydrogel precursor may be incorporated into the porous substrate prior to forming the porous substrate. In other embodiments, the first hydrogel precursor may be positioned in the pores of the porous substrate or onto a surface of the porous substrate following formation of the substrate. In yet other embodiments, the porous substrate may be calendered prior to application of the first hydrogel precursor thereby allowing the first precursor to penetrate into openings on the substrate which were created by the calendaring process. In still other embodiments, the first hydrogel precursor may be applied to the porous substrate in solution followed by evaporation or lyophilization of the solvent. In embodiments, the first hydrogel precursor may be applied to the porous substrate as a coating on at least one side of the substrate or as a film laminated onto at least one side of the substrate.

The second hydrogel precursor likewise may be applied to the porous substrate using any suitable method known to those skilled in the art, including, but not limited to spraying, brushing, dipping, pouring, laminating, etc. In embodiments, the second hydrogel precursor may be applied as a coating on the substrate in any concentration, dimension and configuration capable of forming a hemostatic implant. In embodiments, the second hydrogel precursor coating may penetrate the pores of the porous substrate. The coating may form a non-porous layer or a porous layer. In embodiments, the second hydrogel precursor may be applied to the porous substrate as a film that is laminated onto at least one side of the substrate.

In embodiments where either the first or second hydrogel precursor forms a non-porous layer, i.e., a film, the thickness of the film may be sufficient to allow for only portions of the hydrogel precursor to react with the other hydrogel precursor before the implant seals a wound. In such embodiments, the remaining unreacted hydrogel film may act as a barrier layer between the wound and the surrounding tissue to prevent the formation of adhesions. In forming the hydrogel implant, the precursors may also impart upon the physiological fluids certain properties, such as anti-adhesion. The physiological fluid hydrogel may also act as a barrier layer between the wound and the surrounding tissue to prevent the formation of adhesions. In embodiments, the porous substrate may further contain non-reactive materials that are known to reduce or prevent adhesions, such as hyaluronic acid and the like. In such embodiments, the non-reactive materials may prevent the formation of adhesions after the first and second hydrogel precursors interact.

In addition to providing hemostasis, the implants may further be use for delivery of a bioactive agent. Thus, in some embodiments, at least one bioactive agent may be combined with either the first hydrogel precursor or the second hydrogel precursor and/or may be separately applied to the porous substrate. The agents may be freely admixed with the precursors or may be tethered to the precursors through any variety of chemical bonds. In these embodiments, the present implant can also serve as a vehicle for delivery of the bioactive agent. The term "bioactive agent", as used herein, is used in its broadest sense and includes any substance or mixture of substances that have clinical use. Consequently, bioactive agents may or may not have pharmacological activity per se, e.g., a dye, or fragrance. Alternatively a bioactive agent could be any agent which provides a therapeutic or prophylactic effect, a compound that affects or participates in tissue growth, cell growth, cell differentiation, an anti-adhesive compound, a compound that may be able to invoke a biological action such as an immune response, or could play any other role in one or more biological processes. It is envisioned that the bioactive agent may be applied to the present implant in any suitable form of matter, e.g., films, powders, liquids, gels and the like.

Examples of classes of bioactive agents which may be utilized in accordance with the present disclosure include anti-adhesives, antimicrobials, analgesics, antipyretics, anesthetics, antiepileptics, antihistamines, anti-inflammatories, cardiovascular drugs, diagnostic agents, sympathomimetics, cholinomimetics, antimuscarinics, antispasmodics, hormones, growth factors, muscle relaxants, adrenergic neuron blockers, antineoplastics, immunogenic agents, immunosuppressants, gastrointestinal drugs, diuretics, steroids, lipids, lipopolysaccharides, polysaccharides, platelet activating drugs, clotting factors and enzymes. It is also intended that combinations of bioactive agents may be used.

Anti-adhesive agents can be used to prevent adhesions from forming between the implantable medical device and the surrounding tissues opposite the target tissue, In addition, anti-adhesive agents may be used to prevent adhesions from forming between the coated implantable medical device and the packaging material. Some examples of these agents include, but are not limited to hydrophilic polymers such as poly(vinyl pyrrolidone), carboxymethyl cellulose, hyaluronic acid, polyethylene oxide, poly vinyl alcohols, and combinations thereof.

Suitable antimicrobial agents which may be included as a bioactive agent in the bioactive coating of the present disclosure include triclosan, also known as 2,4,4'-trichloro-2'-hydroxydiphenyl ether, chlorhexidine and its salts, including chlorhexidine acetate, chlorhexidine gluconate, chlorhexidine hydrochloride, and chlorhexidine sulfate, silver and its salts, including silver acetate, silver benzoate, silver carbonate, silver citrate, silver iodate, silver iodide, silver lactate, silver laurate, silver nitrate, silver oxide, silver palmitate, silver protein, and silver sulfadiazine, polymyxin, tetracycline, aminoglycosides, such as tobramycin and gentamicin, rifampicin, bacitracin, neomycin, chloramphenicol, miconazole, quinolones such as oxolinic acid, norfloxacin, nalidixic acid, pefloxacin, enoxacin and ciprofloxacin, penicillins such as oxacillin and pipracil, nonoxynol 9, fusidic acid, cephalosporins, and combinations thereof. In addition, antimicrobial proteins and peptides such as bovine lactoferrin and lactoferricin B may be included as a bioactive agent in the bioactive coating of the present disclosure.

Other bioactive agents which may be included as a bioactive agent in the coating composition applied in accordance with the present disclosure include: local anesthetics; non-steroidal antifertility agents; parasympathomimetic agents; psychotherapeutic agents; tranquilizers; decongestants; sedative hypnotics; steroids; sulfonamides; sympathomimetic agents; vaccines; vitamins; antimalarials; anti-migraine agents; anti-parkinson agents such as L-dopa; anti-spasmodics; anticholinergic agents (e.g., oxybutynin); antitussives; bronchodilators; cardiovascular agents such as coronary vasodilators and nitroglycerin; alkaloids; analgesics; narcotics such as codeine, dihydrocodeinone, meperidine, morphine and the like; non-narcotics such as salicylates, aspirin, acetaminophen, d-propoxyphene and the like; opioid receptor antagonists, such as naltrexone and naloxone; anti-cancer agents; anti-convulsants; anti-emetics; antihistamines; anti-inflammatory agents such as hormonal agents, hydrocortisone, prednisolone, prednisone, non-hormonal agents, allopurinol, indomethacin, phenylbutazone and the like; prostaglandins and cytotoxic drugs; chemotherapeutics, estrogens; antibacterials; antibiotics; anti-fungals; anti-virals; anticoagulants; anticonvulsants; antidepressants; antihistamines; and immunological agents.

Other examples of suitable bioactive agents which may be included in the coating composition include viruses and cells, peptides, polypeptides and proteins, analogs, muteins, and active fragments thereof, such as immunoglobulins, antibodies, cytokines (e.g., lymphokines, monokines, chemokines), blood clotting factors, hemopoietic factors, interleukins (IL-2, IL-3, IL-4, IL-6), interferons (β-IFN, (α-IFN and γ-IFN), erythropoietin, nucleases, tumor necrosis factor, colony stimulating factors (e.g., GCSF, GM-CSF, MCSF), insulin, anti-tumor agents and tumor suppressors, blood proteins, fibrin, thrombin, fibrinogen, synthetic thrombin, synthetic fibrin, synthetic fibrinogen, gonadotropins (e.g., FSH, LH, CG, etc.), hormones and hormone analogs (e.g., growth hormone), vaccines (e.g., tumoral, bacterial and viral antigens); somatostatin; antigens; blood coagulation factors; growth factors (e.g., nerve growth factor, insulin-like growth factor); bone morphogenic proteins, TGF-B, protein inhibitors, protein antagonists, and protein agonists; nucleic acids, such as antisense molecules, DNA, RNA, RNAi; oligonucleotides; polynucleotides; and ribozymes.

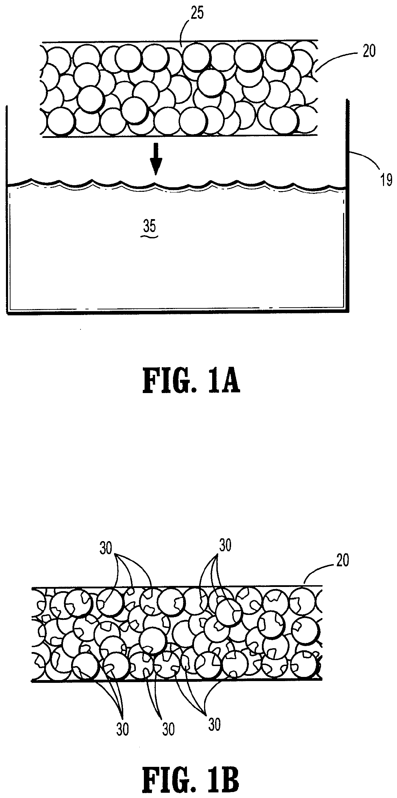

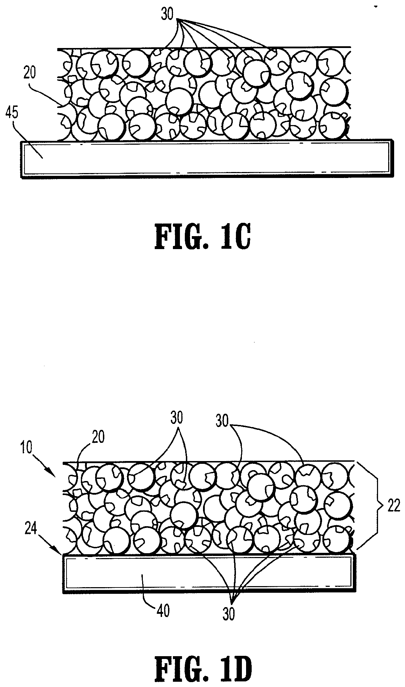

Turning now to Figures 1A - D, a sequence is shown wherein a first hydrogel precursor is applied within the pores of a porous substrate and a second hydrogel precursor is applied to a second portion of the porous substrate. In Fig. 1A, porous substrate 20 is a foam having a plurality of pores 25 defined therein. Solution 35, which includes a first hydrogel precursor dissolved in a solvent, is stored in container 19. Porous substrate 20 is dipped into and completely submerged within solution 35. Upon removal, the implant is dried, removing the solvent from solution 35 and depositing particles that include the first hydrogel precursor 30 within pores 25 of substrate 20, as shown in Fig. IB.

In Fig. 1C, porous substrate 20 containing the first hydrogel precursor is contacted with a melt 45 of the second hydrogel precursor. Upon cooling, the melt 45 of the second hydrogel precursor will solidify to form a film 40 over at least a portion of substrate 20. After application of the film 40 of the second precursor, the implant may be trimmed to any desired size and shape. Implant 10 of Fig. ID is shown having a first hydrogel precursor in the form of particles 30 applied to a first portion 22 of the porous substrate 20 and a second hydrogel precursor in the form of a film 40 applied to a second portion 24 of the porous substrate 20.

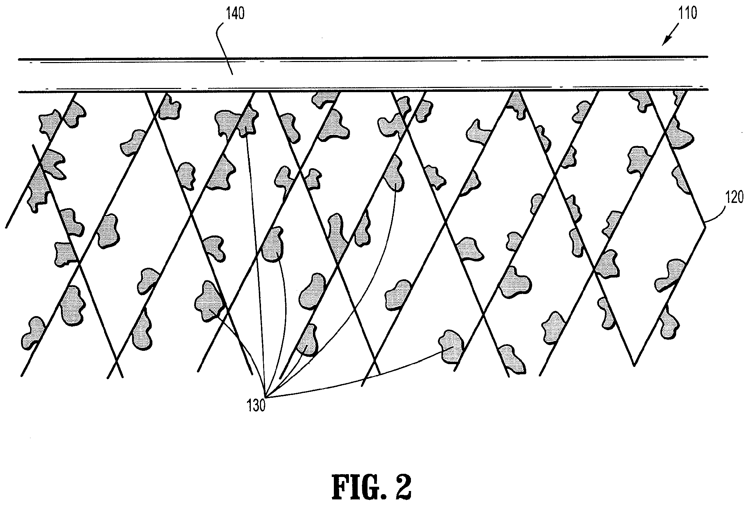

Implant 110 of Fig. 2 is prepared in a manner similar to that show in the sequence of Figures 1A-D, with the exception that the porous substrate 120 is a mesh material having a first hydrogel precursor in the form of particles 130 and a second hydrogel precursor in the form of a film 140 applied thereto. It is contemplated that a non-woven material (not shown) may be used as the porous substrate instead of the foam shown in Figures lA-D or the mesh shown in Figure 2.

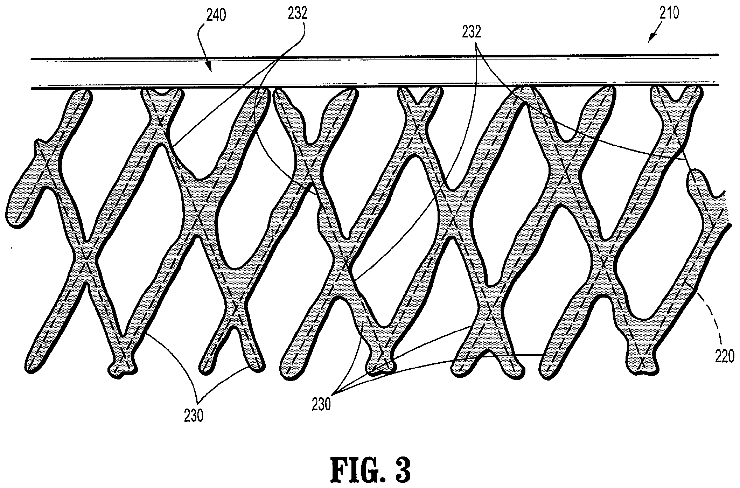

Implant 210 of Fig. 3 is prepared in a manner similar to that shown in the sequence of Figures 1A-D, with the exception that the porous substrate 220 is a mesh material having a first hydrogel precursor in the form of a coating 230 and a second hydrogel precursor in the form of a film 240 applied thereto. Coating 230 of the first hydrogel precursor may be formed by immersing porous substrate 220 into a solution of the first hydrogel precursor or into a melt of the first hydrogel precursor. Alternatively, the first hydrogel precursor may be combined with a film-forming polymer prior to application to the substrate to provide coating 230. Those skilled in the art reading this disclosure will envision other method and materials for applying a coating containing the first hydrogel precursor to the substrate.

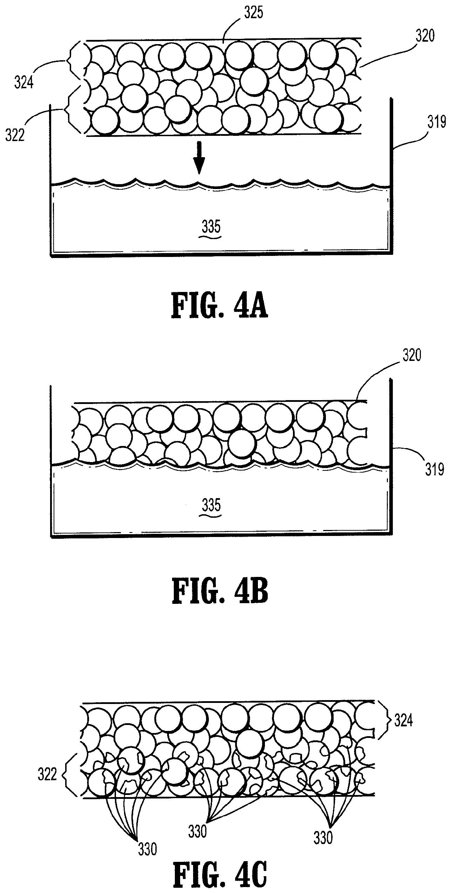

Turning now to Figs. 4A-4C, a sequence is shown wherein a first hydrogel precursor is applied to a first portion of a porous substrate. In Fig. 4A, porous substrate 320 is a foam material having a plurality of pores 325 defined therein, which includes at least a first portion 322 and a second portion 324. Solution 335, which includes a first hydrogel precursor dissolved in a solvent, is stored in container 319. Porous substrate 320 is positioned over solution 335 with first portion 322 facing solution 335 and second portion 324 facing away from solution 335.

In Fig. 4B, first portion 322 of porous substrate 320 is partially submerged in solution 335 by moving porous substrate 320 in the direction of solution 335, as represented by the arrow in Fig. 4A. Only first portion 322 of porous substrate 320 comes in contact with solution 335 so that a sufficient amount of solution 335 may be applied to and fill the pores 325 of first portion 322 of porous substrate 320. Upon removal, the implant is dried, removing the solvent from solution 335 and depositing particles that include the first hydrogel precursor 330 in first portion 322, as shown in Fig. 4C. Particles 330 include the first hydrogel precursor in a dry format and are limited spatially to first portion 322.

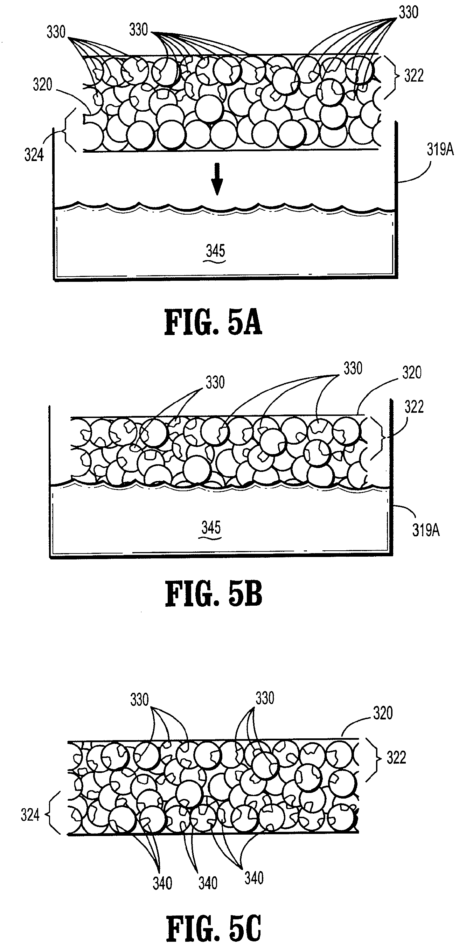

In Figs. 5A-5C, a sequence is shown wherein solution 345 containing a second hydrogel precursor dissolved in a solvent is applied to second portion 324 of porous substrate 320, wherein particles 330 containing a first hydrogel precursor have been previously incorporated into first portion 322 of substrate 320 (See Figs. 4A-4C). Porous substrate 320 is positioned over solution 345 with second portion 324 facing solution 345 and first portion 322 facing away from solution 345.

As shown in Fig. 5B, second portion 324 of porous substrate 320 is partially submerged in solution 345 by moving porous substrate 320 in the direction of solution 345, as represented by the arrow in Fig. 5A. Only second portion 324 of porous substrate 320 comes in contact with solution 345 so that a sufficient amount of solution 345 may be applied to second portion 324. Upon removal, the implant is dried to deposit second particles 40 including the second hydrogel precursor in second portion 324. Particles 340 include the second hydrogel precursor in a dry format and are limited spatially to second portion 324. Porous substrate 320 of Fig. 5C is shown having a first hydrogel precursor applied to a first portion of the substrate and a second hydrogel precursor applied to a second portion of the porous substrate with the first portion of the substrate being spatially separated from the second portion of the porous substrate.

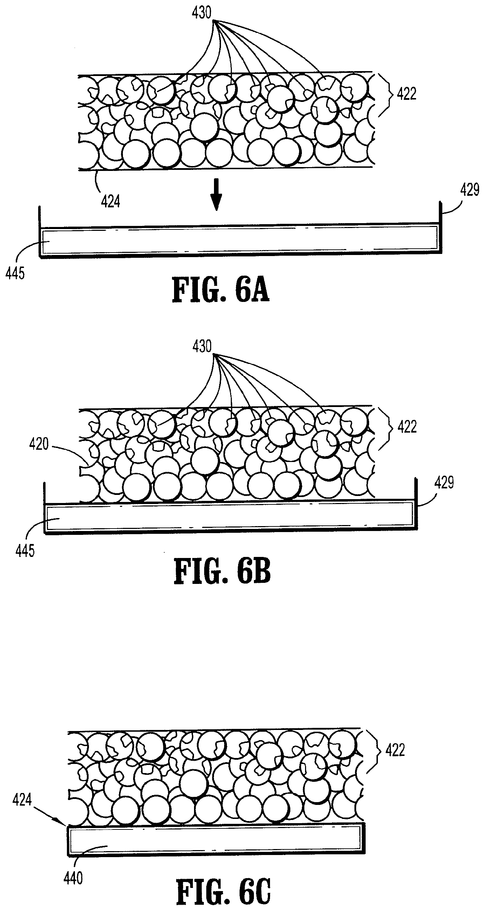

In alternative embodiments, the first and second hydrogel precursors may be applied to the implant in different forms. For example, in Figs. 6A-6C, porous substrate is shown including particles 430 including the first hydrogel precursor applied to first portion 422 with second portion 424 facing a film-forming solution 445A containing the second hydrogel precursor that has been applied to a support 429.

In Fig. 6B, second portion 424 of porous substrate 420 is contacted with and/or partially submerged in film-forming solution 445 by moving porous substrate 420 in the direction of shown by the arrow in Fig. 6A. Only second portion 424 of porous substrate 420 comes in contact with film-forming solution 445 so that a sufficient amount of material 445 may be applied to second portion 424. Film-forming solution 445 is allowed solidify (with or without the application of heat) to form a film over at least a portion of second portion 424. Porous substrate 420 of Fig. 6C is shown having a first hydrogel precursor in the form of particles applied to a first portion of the substrate and a second hydrogel precursor in the form of a film applied to a second portion of the porous substrate with the first portion of the substrate being spatially separated from the second portion of the porous substrate.

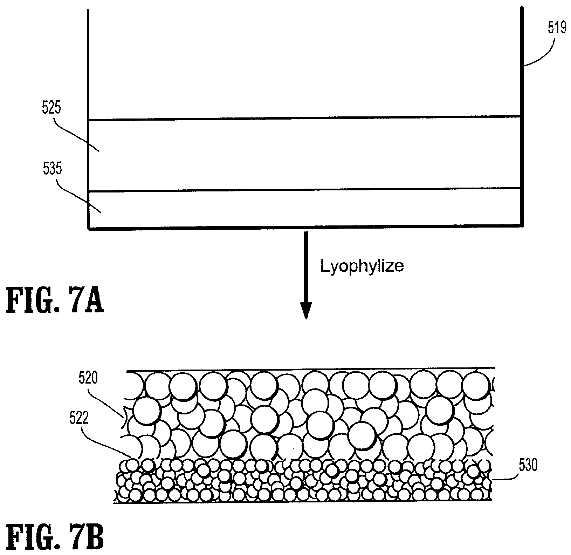

Turning now to Figs. 7A-7B, the porous substrate and a porous layer including the first hydrogel precursor are shown formed together. In Fig. 7A, container 519 includes first solution 525 destined to form the porous substrate and a second solution 535 including the first hydrogel precursor, wherein the two solutions remain substantially as separate layers. The two solutions are lyophilized using any method known to those skilled in the art to form a porous substrate as shown in Fig. 7B, which includes first porous substrate 520, made from the lyophilized first solution 525, connected to a second porous layer 530, made from the lyophilized second solution 535. Second porous layer 530 contains the first hydrogel precursor and is bonded to first porous substrate 520 via first portion 522 to form an implant having two layers of porous material.

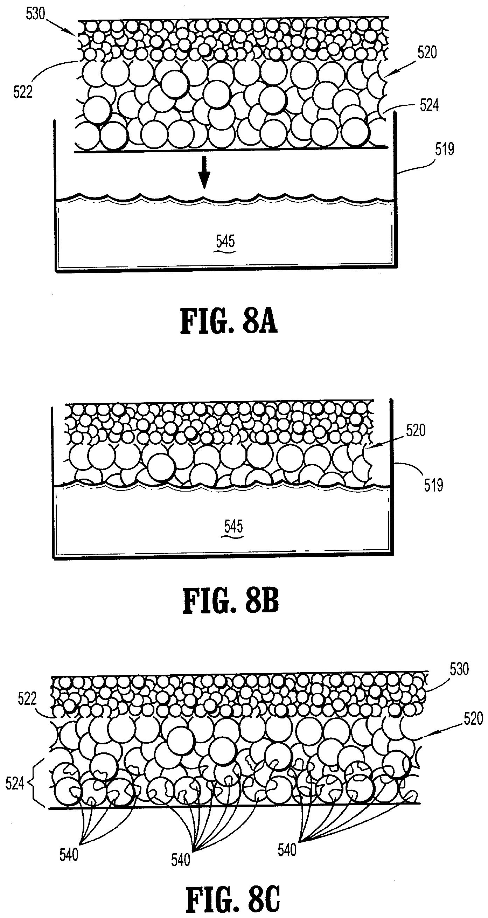

In Figs. 8A-8C, a sequence is shown wherein solution 545 containing a second hydrogel precursor is applied to second portion 524 of porous substrate 520 already having porous substrate 530 including the first hydrogel precursor bonded thereto porous at first portion 522. Porous substrate 520 is positioned over solution 545 with second portion 524 facing solution 545 and first portion 522 and second porous layer 530 facing away from solution 545.

As shown in Fig. 8B, second portion 524 of porous substrate 520 is partially submerged in solution 545 having the first hydrogel precursor dissolved in a solvent by moving porous substrate 520 in the direction of solution 545, as represented by the arrow in Fig. 8A. Only second portion 524 of porous substrate 520 comes in contact with solution 545 so that a sufficient amount of solution 545 may be applied to second portion 524. Upon removal, the implant is dried or allowed to dry to remove the solvent and deposit particles 540 in second portion 524. Second particles 540 include the second hydrogel precursor in a dry format and are limited spatially to second portion 524. Porous substrate 520 of Fig. 8C is shown having a first hydrogel precursor in the form of a foam applied to a first portion of the substrate and a second hydrogel precursor in the form of particles applied to a second portion of the porous substrate with the first portion of the substrate being spatially separated from the second portion of the porous substrate.

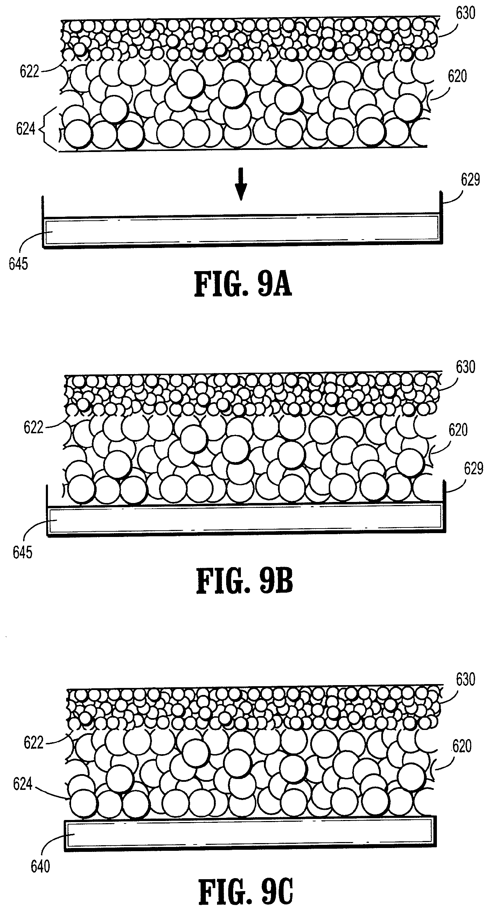

In an alternative embodiment, the porous substrate as shown in Fig. 7B may be combined with a film-forming material including the second hydrogel precursor. As shown in Figs. 9A-9C, porous substrate 620 includes a first portion 622 and a second portion 624, wherein a second porous layer 630 containing a first hydrogel precursor is connected to porous substrate 620 at first portion 622. Second portion 624 is shown facing a film-forming solution 645 applied to support 629. Film-forming material 645 includes a second hydrogel precursor and a solvent.

In Fig. 9B, second portion 624 of porous substrate 620 is contacted with and/or partially submerged in film-forming solution 645 by moving porous substrate 620 in the direction of represented by the arrow in Fig. 9A. Only second portion 624 of porous substrate 620 comes in contact with film-forming solution 645 so that a sufficient amount of material 645 may be applied to second portion 624. Film-forming solution 645 is allowed to form a film over at least a portion of second portion 624. Porous substrate 620 of Fig. 9C is shown having a first hydrogel precursor in the form of a foam applied to a first portion of the substrate and a second hydrogel precursor in the form of a film applied to a second portion of the porous substrate with the first portion of the substrate being spatially separated from the second portion of the porous substrate.

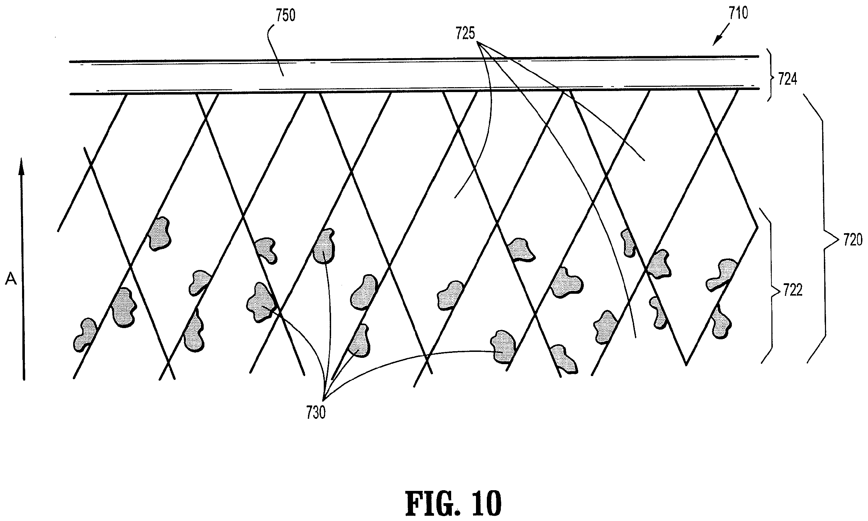

It should be understood that rather than a foam, as shown in Figures 4 - 9, the porous substrate may be a fibrous structure. Thus, in embodiments, and as shown schematically in Figures 10 - 12, the porous substrate may be a fibrous structure, i.e., a woven or non-woven structure. The first and second hydrogel precursors can be applied to a fibrous porous substrate using substantially the same techniques described above with respect to foam porous substrate 20. Accordingly, as with the foam porous substrates described above, where the porous substrate is fibrous, the first and/or second hydrogel precursors may be applied, for example as particles deposited from a solution, non-porous films formed by drying a film-forming solution, or as a foam applied to at least a portion of the fibrous porous substrate. As shown in Fig. 10, for example, implant 710 includes knitted porous substrate 720 including a plurality of pores 725 defined therein and having first portion 722 and second portion 724. Particles 730 containing a first hydrogel precursor in a dry format are applied to first portion 722 in a manner substantially similar to the manner shown above with respect to foam porous substrate 20, above in Figures 4A-C, for example. Film 750 containing a second hydrogel precursor is applied to second portion 724 in a manner substantially similar to the manner shown above with respect to foam porous substrate 720, above in Figures 5A-C, for example. Upon implantation, second portion 750 is applied to tissue in need of hemostasis. Upon contact with tissue, physiological fluids will penetrate implant 710 and migrate in the direction represented by arrow A thereby interacting with and liquefying film 750 before reaching particles 730. It is envisioned that as the fluids are wicked towards first portion 722 of substrate 720, a solution of film 750 will come in contact with particles 730 which will also be dissolved by and mix with the physiologic fluids. This mixing will activate the first and second precursors and allow them to interact and crosslink to form a seal assisting in the hemostatic function of the implant. In embodiments, this newly formed hydrogel/physiological fluid implant will also act as an adhesion barrier.

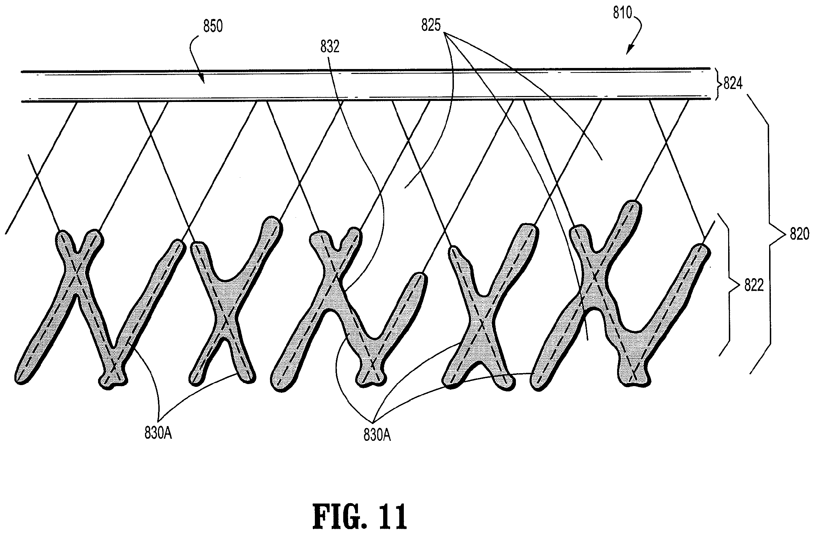

It is further contemplated that the first and/or second hydrogel precursor may be applied from a melt containing the first and/or second hydrogel precursor rather than from a solution. In Fig. 11, for example, implant 810 includes a knitted porous substrate 820 having first portion 822 and second portion 824 wherein second portion 824 again includes film 850 which contains a second hydrogel precursor. In this embodiment, however, the first hydrogel precursor 830 is applied as a coating to first portion 822 from a melt rather than as particles from a solution. As shown, melt 830 essentially coats at least a portion of the fibers of first portion 822 of substrate 820 while allowing pores 825 to remain sufficiently open to allow the migration of fluids through porous substrate 820. It should be understood that the coating 830 may be discontinuous, leaving portions 832 of the substrate 820 may uncoated.

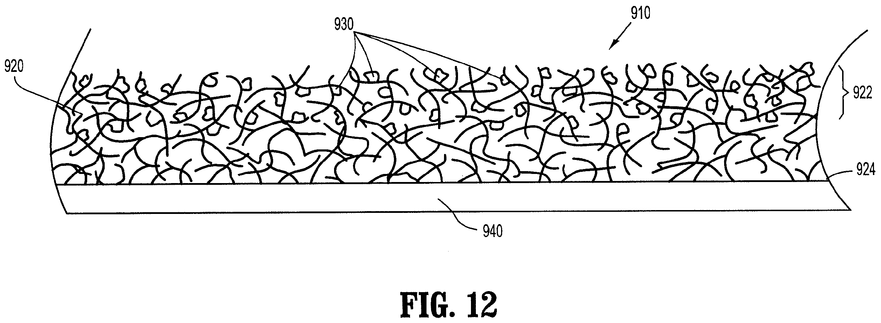

As noted above, the porous substrate may be a non-woven fibrous porous substrate. In Fig. 12, for example, implant 910 is shown as a non-woven porous substrate 920 having a first portion 922 and second portion 924 wherein particles 930 including the first hydrogel precursor applied to first portion 922 and a film 940 including the second hydrogel precursor applied to second portion 924.

### EXAMPLE

A saturated borate buffer solution of trilysine is prepared. The solution contains 20.6 milligrams of trilysine per milliliter of solution. The pH of the solution is about 9.2. A sheet of oxidized cellulose is dipped into the solution and then fixed to a rack for drying. The rack is placed into a vacuum oven. The oven is pumped down to about 6.67 Pa (50 mTorr) and kept at a temperature of about 25°C for about three days to reduce the moisture level to less than 2% by weight. An eight arm N-hydroxysuccinimidyl-functionalized polyethylene glycol having a molecular weight of about fifteen thousand is melted at about 50° C. on a hot plate. The dried trilysine-containing oxidized cellulose sheet is placed into contact with the melted PEG component. After cooling, the PEG component forms a film on one side of the implant.

The resulting product is trimmed to a 5.08 cm by 5.08 cm square (a 2 inch by 2 inch square), dried and packaged in a foil container.

In use, the foil package is opened and the implant is applied to a bleeding wound with the PEG film side against the wound. Within seconds, hemostasis occurs.

It will be understood that various modifications may be made to the embodiments disclosed herein. For example, more than two precursors may be applied to the porous substrate to form the hemostatic implant. As another example, the first and second precursors may each be applied to the porous substrate as a film.

## Claims

1. An implant for use as a hemostat comprising:
a porous fibrous substrate having pores over at least a portion thereof;
a first hydrogel precursor applied to a first portion of the porous substrate; and
a second hydrogel precursor applied to a second portion of the porous substrate,
the first portion of the porous substrate being spatially separated from the second portion of the porous substrate,
wherein the first hydrogel precursor and the second hydrogel precursor react to form a hydrogel when exposed to physiologic fluid.

2. The implant of claim 1, wherein the second hydrogel precursor is applied as a film.

3. The implant of claim 1 or claim 2, wherein the porous substrate is a knitted textile.

4. The implant of claim 1 or claim 2, wherein the porous substrate is a non-woven textile.

5. The implant of any preceding claim, wherein the porous substrate is made from a bioabsorbable material.

6. The implant of any of claims 1 to 4, wherein the porous substrate is made from a non-bioabsorbable material.

7. The implant of any preceding claim, wherein the porous substrate is made from a combination of natural, synthetic, bioabsorbable and non-bioabsorbable materials.

8. The implant of any preceding claim, wherein the porous substrate comprises, but is not limited to poly(lactic acid), poly (glycolic acid), poly(lactide, poly(glycolide), poly(trimethylene carbonate), poly (dioxanone), poly (hydroxybutyrate), poly (phosphazine), polyesters, polyethylene terephthalate, ultra-high molecular weight polyethylene, polyethylene glycols, polyethylene oxides, polyacrylamides, polyhydroxyethylmethylacrylate, polyvinylpyrrolidone, polyvinyl alcohols, polyacrylic acid, polyacetate, polycaprolactone, polypropylene, aliphatic polyesters, glycerols, poly(amino acids), copoly (ether-esters), polyalkylene oxalates, poly (saccharides), polyamides, poly (iminocarbonates), polyalkylene oxalates, polyoxaesters, polyorthoesters, polyphosphazenes, biopolymers, polymer drugs and copolymers, block copolymers, homopolymers, blends or combinations thereof.

9. The implant of any of claims 1 to 5, wherein the porous substrate is made from oxidized cellulose.

10. The implant of any preceding claim, wherein the first hydrogel precursor comprises particles, a foam or a film.

11. The implant of any preceding claim, wherein the second hydrogel precursor comprises particles, a foam or a film.

12. The implant of any preceding claim, further comprising at least one bioactive agent.

13. The implant of claim 12, wherein at least one bioactive agent is combined with either the first hydrogel precursor or the second hydrogel precursor and/or is separately applied to the porous substrate.

14. The implant of claim 12 or claim 13, wherein the at least one bioactive agent comprises anti-adhesives, antimicrobials, analgesics, antipyretics, anesthetics, antiepileptics, antihistamines, anti-inflammatories, cardiovascular drugs, diagnostic agents, sympathomimetics, cholinomimetics, antimuscarinics, antispasmodics, hormones, growth factors, muscle relaxants, adrenergic neuron blockers, antineoplastics, immunogenic agents, immunosuppressants, gastrointestinal drugs, diuretics, steroids, lipids, lipopolysaccharides, polysaccharides, platelet activating drugs, clotting factors and enzymes or combinations thereof.

15. The implant of any of claims 12, 13 or 14 wherein the at least one bioactive agent comprises 2,4,4'-trichloro-2'-hydroxydiphenyl ether, chlorhexidine and its salts, including chlorhexidine acetate, chlorhexidine gluconate, chlorhexidine hydrochloride, and chlorhexidine sulfate, silver and its salts, including silver acetate, silver benzoate, silver carbonate, silver citrate, silver iodate, silver iodide, silver lactate, silver laurate, silver nitrate, silver oxide, silver palmitate, silver protein, and silver sulfadiazine, polymyxin, tetracycline, aminoglycosides, such as tobramycin and gentamicin, rifampicin, bacitracin, neomycin, chloramphenicol, miconazole, quinolones such as oxolinic acid, norfloxacin, nalidixic acid, pefloxacin, enoxacin and ciprofloxacin, penicillins such as oxacillin and pipracil, nonoxynol 9, fusidic acid, cephalosporins, antimicrobial proteins and peptides such as bovine lactoferrin and lactoferricin B, or combinations thereof.

## Patentansprüche

1. Implantat zur Verwendung als Hämostat, welches umfasst:
ein poröses Fasersubstrat mit Poren über wenigstens einem Teil davon;
eine erste Hydrogel-Vorstufe, die auf einen ersten Teil des porösen Substrats aufgetragen ist; und
eine zweite Hydrogel-Vorstufe, die auf einen zweiten Teil des porösen Substrats aufgetragen ist,
wobei der erste Teil des porösen Substrats von dem zweiten Teil des porösen Substrats räumlich getrennt ist,
wobei die erste Hydrogel-Vorstufe und die zweite Hydrogel-Vorstufe reagieren, um ein Hydrogel zu bilden, wenn sie einem physiologischen Fluid ausgesetzt werden.

2. Implantat nach Anspruch 1, wobei die zweite Hydrogel-Vorstufe als Film aufgetragen ist.

3. Implantat nach Anspruch 1 oder Anspruch 2, wobei das poröse Substrat ein gestricktes Textil ist.

4. Implantat nach Anspruch 1 oder Anspruch 2, wobei das poröse Substrat ein Vliesstoff ist.

5. Implantat nach einem vorstehenden Anspruch, wobei das poröse Substrat aus einem bioabsorbierbaren Material besteht.

6. Implantat nach einem der Ansprüche 1 bis 4, wobei das poröse Substrat aus einem nicht bioabsorbierbaren Material besteht.

7. Implantat nach einem vorstehenden Anspruch, wobei das poröse Substrat aus einer Kombination von natürlichen, synthetischen, bioabsorbierbaren und nicht bioabsorbierbaren Materialien besteht.

8. Implantat nach einem vorstehenden Anspruch, wobei das poröse Substrat Poly(milchsäure), Poly(glykolsäure), Poly(lactid), Poly(glykolid), Poly(trimethylen-carbonat), Poly(dioxanon), Poly(hydroxybutyrat), Poly(phosphazin), Polyester, Polyethylenterephthalat, ultrahochmolekulares Polyethylen, Polyethylenglykole, Polyethylenoxide, Polyacrylamide, Polyhydroxyethylmethylacrylat, Polyvinylpyrrolidon, Polyvinylalkohole, Polyacrylsäure, Polyacetat, Polycaprolacton, Polypropylen, aliphatische Polyester, Glycerine, Poly(aminosäuren), Copoly (Etherester), Polyalkylenoxalate, Poly(saccharide), Polyamide, Poly(iminocarbonate), Polyalkylenoxalate, Polyoxaester, Polyorthoester, Polyphosphazene, Bipolymere, polymere Arzneimittel und Kopolymere, Blockkopolymere, Homopolymere, Mischungen oder Kombinationen davon umfasst, jedoch nicht darauf beschränkt ist.

9. Implantat nach einem der Ansprüche 1 bis 5, wobei das poröse Substrat aus oxidierter Cellulose besteht.

10. Implantat nach einem vorstehenden Anspruch, wobei die erste Hydrogel-Vorstufe Partikel, einen Schaumstoff oder einen Film umfasst.

11. Implantat nach einem vorstehenden Anspruch, wobei die zweite Hydrogel-Vorstufe Partikel, einen Schaumstoff oder einen Film umfasst.

12. Implantat nach einem vorstehenden Anspruch, weiter umfassend mindestens einen bioaktiven Wirkstoff.

13. Implantat nach Anspruch 12, wobei mindestens ein bioaktiver Wirkstoff entweder mit der ersten Hydrogel-Vorstufe oder der zweiten Hydrogel-Vorstufe kombiniert ist und/oder separat auf das poröse Substrat aufgetragen ist.

14. Implantat nach Anspruch 12 oder Anspruch 13, wobei der mindestens eine bioaktive Wirkstoff Antihaftmittel, Antimikrobika, Analgetika, Antipyretika, Anästhetika, Antiepileptika, Antihistaminika, Entzündungshemmer, Herz-Kreislauf-Arzneimittel, Diagnostika, Sympathomimetika, Cholinomimetika, Antimuskarinika, Antispasmodika, Hormone, Wachstumsfaktoren, Muskelrelaxantien, adrenerge Neuronenblocker, Antineoplastika, immunogene Wirkstoffe, Immunsuppressiva, Magen-Darm-Arzneimittel, Diuretika, Steroide, Lipide, Lipopolysaccharide, Polysaccharide, plättchenaktivierende Arzneimittel, Gerinnungsfaktoren und Enzyme oder Kombinationen davon umfasst.

15. Implantat nach einem der Ansprüche 12, 13 oder 14, wobei der mindestens eine bioaktive Wirkstoff 2,4,4'-Trichlor-2'-hydroxydiphenylether, Chlorhexidin und seine Salze, einschließlich Chlorhexidinacetat, Chlorhexidingluconat, Chlorhexidinhydrochlorid und Chlorhexidinsulfat, Silber und seine Salze, einschließlich Silberacetat, Silberbenzoat, Silbercarbonat, Silbercitrat, Silberjodat, Silberjodid, Silberlaktat, Silberlaurat, Silbernitrat, Silberoxid, Silberpalmitat, Silberprotein und Silbersulfadiazin, Polymyxin, Tetracyclin, Aminoglykoside wie Tobramycin und Gentamicin, Rifampicin, Bacitracin, Neomycin, Chloramphenicol, Miconazol, Chinolone wie Oxolinsäure, Norfloxacin, Nalidixinsäure, Pefloxazin, Enoxazin und Ciprofloxazin, Penizilline wie Oxacillin und Pipracil, Nonoxynol 9, Fusidinsäure, Cephalosporine, antimikrobielle Proteine und Peptide wie Rinderlaktoferrin und Lactoferricin B oder Kombinationen davon umfasst.

## Revendications

1. Implant pour usage comme hémostat comprenant :
un substrat fibreux poreux ayant des pores sur au moins une portion de celui-ci ;
un premier précurseur d'hydrogel appliqué à une première portion du substrat poreux ; et
un second précurseur d'hydrogel appliqué à une seconde portion du substrat poreux,
la première portion du substrat poreux étant spatialement séparée de la seconde portion du substrat poreux,
dans lequel le premier précurseur d'hydrogel et le second précurseur d'hydrogel réagissent pour former un hydrogel lorsqu'ils sont exposés à du fluide physiologique.

2. Implant selon la revendication 1, dans lequel le second précurseur d'hydrogel est appliqué comme un film.

3. Implant selon la revendication 1 ou la revendication 2, dans lequel le substrat poreux est un textile tricoté.

4. Implant selon la revendication 1 ou la revendication 2, dans lequel le substrat poreux est un textile non tissé.

5. Implant selon l'une quelconque des revendications précédentes, dans lequel le substrat poreux est constitué d'un matériau bioabsorbable.

6. Implant selon l'une quelconque des revendications 1 à 4, dans lequel le substrat poreux est constitué d'un matériau non bioabsorbable.

7. Implant selon l'une quelconque des revendications précédentes, dans lequel le substrat poreux est constitué d'une combinaison de matériaux naturel, synthétique, bioabsorbable et non bioabsorbable.

8. Implant selon l'une quelconque des revendications précédentes, dans lequel le substrat poreux comprend, mais sans s'y limiter, de l'acide polylactique, de l'acide polyglycolique, du poly(lactide), du poly(glycolide), du poly(carbonate de triméthylène), du poly(dioxanone), du poly(hydroxybutyrate), de la poly(phosphazine), des polyesters, du polyéthylène téréphtalate, du polyéthylène à poids moléculaire très élevé, des polyéthylène glycols, des oxydes de polyéthylène, des polyacrylamides, du polyhydroxyéthylméthylacrylate, de la polyvinylpyrrolidone, des alcools polyvinyliques, un acide polyacrylique, du polyacétate, du polycaprolactone, du polypropylène, des polyesters aliphatiques, des glycérols, des acides polyaminés, des copoly(éther-esters), des oxalates de polyalkylène, des poly(saccharides), des polyamides, des poly(iminocarbonates), des oxalates de polyalkylène, des polyoxaesters, des polyorthoesters, des polyphosphazènes, des biopolymères, des médicaments polymères et copolymères, des copolymères séquencés, des homopolymères, des mélanges et combinaisons de ceux-ci.

9. Implant selon l'une quelconque des revendications 1 à 5, dans lequel le substrat poreux est constitué de cellulose oxydée.

10. Implant selon l'une quelconque des revendications précédentes, dans lequel le premier précurseur d'hydrogel comprend des particules, une mousse ou un film.

11. Implant selon l'une quelconque des revendications précédentes, dans lequel le second précurseur d'hydrogel comprend des particules, une mousse ou un film.

12. Implant selon l'une quelconque des revendications précédentes, comprenant en outre au moins un agent bioactif.

13. Implant selon la revendication 12, dans lequel au moins un agent bioactif est combiné avec l'un ou l'autre du premier précurseur d'hydrogel et du second précurseur d'hydrogel et/ou est appliqué séparément sur le substrat poreux.

14. Implant selon la revendication 12 ou la revendication 13, dans lequel l'au moins un agent bioactif comprend des antiadhésifs, des antimicrobiens, des analgésiques, des antipyrétiques, des anesthésiques, des antiépileptiques, des antihistaminiques, des anti-inflammatoires, des médicaments cardiovasculaires, des agents de diagnostic, des sympathomimétiques, des cholinomimétiques, des antimuscariniques, des antispasmodiques, des hormones, des facteurs de croissance, des relaxants musculaires, des agents anti-adrénergiques, des antinéoplasiques, des agents immunogènes, des immunosuppresseurs, des médicaments gastro-intestinaux, des diurétiques, des stéroïdes, des lipides, des lipopolysaccharides, des polysaccharides, des médicaments activateurs de plaquettes, des facteurs de coagulation et des enzymes ou des combinaisons de ceux-ci.

15. Implant selon l'une quelconque des revendications 12, 13 ou 14, dans lequel l'au moins un agent bioactif comprend de l'éther 2,4,4'-trichloro-2'-hydroxydiphényle, de la chlorhexidine et ses sels, y compris, l'acétate de chlorhexidine, le glyconate de chlorhexidine, l'hydrochlorure de chlorhexidine et le sulfate de chlorhexidine, de l'argent et ses sels, y compris, l'acétate d'argent, le benzoate d'argent, le carbonate d'argent, le citrate d'argent, l'iodate d'argent, l'iodure d'argent, le lactate d'argent, le laurate d'argent, le nitrate d'argent, l'oxyde d'argent, le palmitate d'argent, la protéine d'argent et la sulfadiazine d'argent, de la polymyxine, de la tétracycline, des aminoglycosides, tels que la tobramycine et la gentamicine, de la rifampicine, de la bacitracine, de la néomycine, du chloramphénicol, du miconazole, des quinolones tels que l'acide oxolinique, la norfloxacine, l'acide nalidixique, la péfloxacine, l'énoxacine et la ciprofloxacine, des pénicillines telles que l'oxacilline et le pipracil, du nonoxynol 9, de l'acide fusidique, des céphalosporines, des protéines antimicrobiennes et des peptides telles que la lactoferrine bovine et la lactoferrine B, ou des combinaisons de ceux-ci.
